# EUROPEAN PATENT APPLICATION

(11) **EP 4 324 453 A1**
(43) Date of publication of application: **21.02.2024**
(21) Application number: 23165434.4
(22) Date of filing: 30.03.2023
(51) Int. Cl.: A61K 8/44, A61K 8/67, A61K 8/92, A61Q 5/00, A61Q 5/12

(54) **COMPOSITION OF COSMETIC CEMENT FOR HAIR RECONSTRUCTION**

(30) Priority: 30.03.2022 PL 44079722
(71) Applicant: Aflofarm Farmacja Polska SP. Z O.O., 95-200 Pabianice (PL)
(72) Inventor: Wahl, Hanna, 95-200 Pabianice (PL); Swiatczak, Elzbieta, 95-200 Pabianice (PL); Dabrowa, Marek, 90-146 Lódz (PL); Gil, Katarzyna, 97-200 Tomaszów Mazowiecki (PL); Daszkiewicz, Malgorzata, 92-328 Lódz (PL); Ochocki, Marcin, 92-737 Lódz (PL); Pasinski, Jaroslaw, 91-035 Lódz (PL); Madejczyk, Arkadiusz, 95-054 Ksawerów (PL); Pawlikowski, Lukasz, 95-200 Pabianice (PL)
(74) Representative: Witek, Rafal

(57) **Abstract**

The invention relates to a cosmetic cement composition for hair restoration, characterized in that it contains 1.5% of conditioner I (% by weight, amount per 100 g of the product), 1.33% by weight of conditioner II, 0.5% by weight of conditioner III, 0.2% to 0.3% by weight of conditioner IV, 4% to 6% by weight of emulsifier I, 3% to 4% by weight of viscosity adjuster I, 1% to 4% by weight of antistatic I, 2% by weight of softener, 1% by weight of emulsifier II, 1% by weight of hair nourishing agent, 0.5% by weight of vitamin E acetate, 0.2% by weight of viscosity adjuster II, 0.05% by weight of disodium edetate, preferably the composition contains 1.5% by weight of conditioner VII, preferably the composition contains 0.29% to 2% by weight of pH adjuster, preferably the composition contains 1% by weight of antistatic II, preferably the composition contains 1.6% by weight of conditioner VI, preferably the composition contains 1% by weight of jojoba oil. The invention further includes a method for obtaining the composition.

## Description

The invention relates to a cosmetic cement composition for hair restoration. The invention also includes a method for obtaining the composition.

From European patent application EP1570833A1 is known a composition in the form of an O/W emulsion, in particular a microemulsion, containing a surfactant, a silicone oil, a mixture of plant extracts (aqueous-alcoholic) from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. The concentration of ceramide in the composition can be in the range of 0.01% to 2%, and especially 0.01% to 1% by weight per total weight of the composition. The fatty acids may be contained in the range of 0.01% to 2.5%, and especially 0.01% to 1% by weight per total weight of the composition.

From European patent description EP1593364B1, a cosmetic composition is known containing three liquid phases which mixes homogenously upon shaking and separates again into three phases in a short period of time. The composition can contain natural oils such as: olive oil, almond oil, avocado oil, weizenkeim oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil. The composition can also contain plant extracts, in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

From French patent FR2975298B1, the use of peptides with a molecular weight of less than 2,000 Daltons from *Cynara scolymus* leaves or hydrolysate of *Cynara scolymus* leaves containing peptides as an active ingredient in a cosmetic composition is known, where the active ingredient is intended to normalise skin thickness, increase hydration and/or smoothen facial wrinkles. The patent describes the preparation of active ingredients, involving aqueous extraction of *Cynara scolymus* leaf powder, enzymatic hydrolysis using protease, separation of soluble and insoluble phases, inactivation of enzymes by heat treatment, recovery of peptides by ultrafiltration with a molecular weight of 2,000 Daltons, filtration, sterilisation of the resulting filtrate and addition of an additive to eliminate phenolic compounds during the process.

A cosmetic composition containing *Cynara scolymus* and *Brassica oleracea* extracts as active ingredients is known from Korean patent application KR20160123732. According to the description, it contains 0.001%-20.0% by weight of *Cynara scolymus* and *Brassica oleracea* var. extracts. Extracts from *Cynara scolymus* and *Brassica oleracea* var. have antioxidant effects, inhibit MMP-1 production, promote collagen biosynthesis, inhibit melanin production, moisturise the skin, soothe skin irritation and alleviate wrinkles. The composition has applications to support the skin regeneration process.

A composition is known from European patent application EP3930673A1, which contains a plant product and a texturing agent. The composition has the use to purify, hydrate and/or enhance the radiance of the skin. The plant product is a pulp or puree of a plant species of the type: beet, carrot, celery root, chervil, chervis, kohlrabi, galangal, ginger, ginseng, greater burdock, helianthi, yam, maceron, cassava, turnip, parsnip, sweet potato, parsley, potato, radish, horseradish, rutabaga, cultivated salsify, Spanish scorson, and/or Jerusalem artichoke.

A scalp care composition containing butyl esters of fatty acids of avocado oil, known as 5-α-avocuta, and one or more thickening polymers is known from European patent description EP1604640B1. It may contain plant extracts. The composition shows, in particular, an excellent anti-fat effect due to the 5-α-avocuta content and, in combination with additional active ingredients, is excellently suitable for use in curing other scalp related cosmetic problems. Plant extracts include, in particular, extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves and white hawthorn. Plant extracts are incorporated in the composition usually in an amount of about 0.01% to about 10%, preferably 0.05% to 7.5%, in particular 0.1% to 5% by weight, calculated as dry residue thereof to the total composition.

Thus, a technical problem would be to provide a cosmetic composition for human use on hair, which would have the effect of unblocking the hair follicle orifices, reducing the splitting and fraying of the hair structure and protecting the hair from damage caused by hairdressing treatments, whereby the composition should also reduce hair static electricity and protect the hair colour from losing its intensity. Another problem is to provide a composition that would solve the above problems, and at the same time capable of being stored without the risk of microbial/fungal infection with no preservative additives.

The subject of the invention is a cosmetic cement composition for hair restoration, characterised in that it contains 1.5% of conditioner I (% by weight, amount per 100 g of the product), 1.33% by weight of conditioner II, 0.5% by weight of conditioner III, 0.2% to 0.3% by weight of conditioner IV, 4% to 6% by weight of emulsifier I, 3% to 4% by weight of viscosity adjuster I, 1% to 4% by weight of antistatic I, 2% by weight of softener, 1% by weight of emulsifier II, 1% by weight of hair nourishing agent, 0.5% by weight of vitamin E acetate, 0.2% by weight of viscosity adjuster II, 0.05% by weight of disodium edetate, preferably the composition contains 1.5% by weight of conditioner VII, preferably the composition contains 0.29% to 2% by weight of pH adjuster, preferably the composition contains 1% by weight of antistatic II, preferably the composition contains 1.6% by weight of conditioner VI, preferably the composition contains 1% by weight of jojoba oil.

In a preferred embodiment of the invention, the composition contains 1.5% of conditioner I (% by weight, amount per 100 g of the product), 1.33% by weight of conditioner II, 0.5% by weight of conditioner III, 0.2% to 0.3% by weight of conditioner IV, 4% to 6% by weight of emulsifier I, 3% to 4% by weight of viscosity adjuster I, 1% to 4% by weight of antistatic I, 2% by weight of softener, 1% by weight of emulsifier II, 1% by weight of hair nourishing agent, 0.5% by weight of vitamin E acetate, 0.2% by weight of viscosity adjuster II, 0.05% by weight of disodium edetate, 1.6% by weight of conditioner VI, preferably the composition contains 0.29% by weight of sodium hydroxide as a pH adjuster, preferably the composition contains 1% by weight of antistatic II.

In a further preferred embodiment of the invention, the composition contains 1.5% of conditioner I (% by weight, amount per 100 g of the product), 1.33% by weight of conditioner II, 0.5% by weight of conditioner III, 0.2% to 0.3% by weight of conditioner IV, 4% to 6% by weight of emulsifier I, 3% to 4% by weight of viscosity adjuster I, 1% to 4% by weight of antistatic I, 2% by weight of softener, 1% by weight of emulsifier II, 1% by weight of hair nourishing agent, 0.5% by weight of vitamin E acetate, 0.2% by weight of viscosity adjuster II, 0.05% by weight of disodium edetate, 1.6% by weight of conditioner VI, 2% by weight of lactic acid solution (60% aqueous solution) as pH adjuster, 1% by weight of antistatic II.

In a further preferred embodiment of the invention, the composition contains a fragrance composition in an amount of 1% by weight of the composition.

In yet another preferred embodiment of the invention, the composition contains water in an amount of up to 100% by weight of the composition.

In yet another preferred embodiment of the invention, the pH adjuster is selected from the group consisting of: 60% (by weight) aqueous solution of sodium lactate or 10% (by weight) aqueous solution of sodium hydroxide.

A second subject of the invention is a method for obtaining the cosmetic cement composition for hair restoration as defined in the first subject of the invention, comprising preparation of an oil phase, an aqueous phase, a combination of the oil phase and the aqueous phase, characterised in that
a) the oil phase is prepared by mixing emulsifier I, viscosity adjuster I, emulsifier II, hair nourishing agent I, and the resulting mixture is heated to a temperature between 75°C and 80°C and stirred,
b) the aqueous phase is prepared by mixing water, disodium edetate, viscosity adjuster II, antistatic I, and the resulting mixture is stirred and heated to a temperature between 75°C and 80°C,
c) the oil phase and the aqueous phase are then combined and the combined phases are homogenised for 5 minutes, and the homogenised mixture is cooled to a temperature between 40° and 42°C and conditioner I, conditioner II, conditioner III, conditioner IV, vitamin E acetate, softener are added, preferably conditioner VII is added, preferably conditioner V is added, preferably conditioner VI is added and the resulting mixture is homogenised for 5 min, preferably the mixture is cooled to a temperature between 25°C and 30°C, preferably a pH adjuster is added.

In a preferred embodiment of the invention in step c), after combining the oil phase and the aqueous phase, the combined phases are homogenised for 5 minutes, and the homogenised mixture is cooled to a temperature between 40°C and 42°C and conditioner I, conditioner II, conditioner III, conditioner IV, vitamin E acetate, conditioner V, softener are added to it and the resulting mixture is homogenised for 5 minutes, and a pH adjuster is added, and the mixture is cooled to a temperature between 25°C and 30°C.

In a further preferred embodiment of the invention in step c), after combining the oil phase and the aqueous phase, the combined phases are homogenised for 5 minutes, and the homogenised mixture is cooled to a temperature between 40° and 42°C and conditioner I, conditioner II, conditioner III, conditioner IV, vitamin E acetate, conditioner V, conditioner VI, softener are added to it and the resulting mixture is homogenised for 5 minutes, a pH adjuster is added, and the mixture is cooled to between 25°C and 30°C.

In a further preferred embodiment of the invention in step c), a pH adjuster is added after homogenisation and before cooling, the pH adjuster being selected from the group comprising: 60% (by weight) sodium lactate aqueous solution or 10% (by weight) sodium hydroxide aqueous solution, preferably 60% (by weight) sodium lactate aqueous solution.

In yet another preferred embodiment of the invention, a fragrance composition is added in step c).

In yet another embodiment of the invention, the mixture in step a) is stirred until the components of the oil phase are dissolved.

In yet another preferred embodiment of the invention, the mixture in step b) is stirred until the components of the aqueous phase are dissolved.

The restorative hair cement has a repairing effect, strengthens and smoothens the hair and improves its elasticity. Biomimetic ceramide, derived from vegetable fatty acids, replenishes the weakened lipid cement, thus rebuilding the hair and protecting it from damage caused by combing and styling. The panthenol contained in the mask acts as a humectant, attracting and retaining moisture in the hair, moisturising and making it more supple. Artichoke extract protects the hair surface, smoothing and disciplining the hair. The complex of oils used in the mask - sunflower, macadamia and monoi - conditions the hair, having a protective effect and improving its appearance. Its combination with quinoa and lemon balm extracts has an antioxidant effect, protecting against the negative effects of free radicals. The rice and corn derivative protects against heat and helps to maintain intense colour. When used regularly, the mask prevents split ends and hair breakage. An improvement in the appearance of the hair is visible after the first use.

The purpose of a conditioner is to smoothen, shine, moisturise and lubricate the skin or hair. The use of a conditioner improves the appearance of the skin or hair. An emulsifier aims to enable the formation of homogeneous mixtures of immiscible liquids by changing the interfacial tension. An antistatic aims to reduce static electricity by neutralising surface electrical charges, e.g. on a hair surface. A viscosity adjuster aims to increase or decrease the viscosity of a cosmetic.

The composition according to the solution is characterised by a number of advantages, such as:
- cleansing follicular orifices of the hair - unblocking follicular orifice results in adequate growth of new hair and proper functioning of the scalp;
- very effective improvement in the condition of hair ends - reducing the splitting and fraying of their structure;
- hair remains fresh for a long time after applying the mask;
- the product reduces hair static electricity;
- the product protects hair colour from losing its intensity;
- the product protects hair from damage, for example during combing and styling.

### Example 1. Development of the restorative hair cement formulation

During the formulation research, a number of technological trials were carried out in order to develop an optimal product formulation that ensures the unique effects of the composition. The initial product composition is shown in Table 1.1. Table 1.1 also identifies the components of the composition in terms of functionality, e.g. dexpanthenol (a commercially available ingredient) acts as a conditioner in the composition. However, each composition contains more than one conditioner, and this has been taken into account by numbering the individual ingredients with the same role as the aforementioned dexpanthenol (conditioner II), e.g. keracyn - conditioner III. The functions indicated in this embodiment - composition P1 (Table 1.1) - for a given ingredient will be identical for the same ingredients in the other example compositions (P2-P6). If a composition contains a new ingredient, it is designated by assigning it a function and possibly a further number if, for a given composition, the same function is performed by an already designated ingredient.

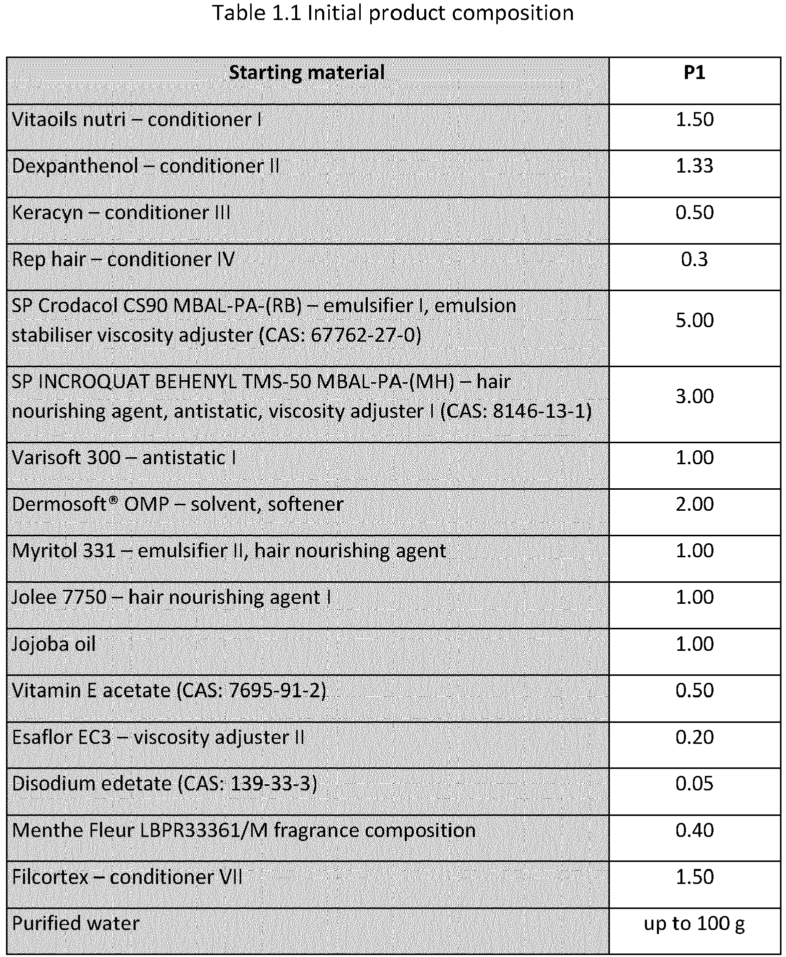

Commercial sources of selected ingredients below:

| | |
|---|---|
| Hydrafeel-3 | https://bccosmetica ndfood.co m/ en/blog/2021/02/01/hyd rafeel-3/ |
| Vitaoils nutri | https://www.weareprovital.com/pl/caremotives/oilycares/nutri-vitaolejki |
| Balm mint extract HGL-MS | https://www.weareprovital.com/pl/caremotives/vitacares/balm-mint-extract-h_gl_---m_s_ |
| Rep'hair | https://www.aston-chemicals.com/pl/single-product?id=1631 |
| Keracyn | https://www.weareprovital.com/pl/careactives/keracyn |
| Filcortex | https://assessa.com.br/en/filcortex/ |
| Myritol 331 | https://carecreations.basf.us/products/myritol-331 |
| Jolee 7750 | https://surfachem.pl/products/jolee-7750 |
| Esaflor EC3 | https://www.knowde.com/stores/lamberti-personal-care/products/lamberti-s-p-a-esaflor-ec-3/ |

Preparation description:
Oil phase: SP Crodacol CS90 MBAL-PA-(RB) (emulsifier I, emulsion stabilizer viscosity adjuster),
SP INCROQUAT BEHENYL TMS-50 MBAL-PA-(MH) (hair nourishing agent, antistatic, viscosity adjuster I), Myritol 331 (emulsifier II, hair nourishing agent), Jolee 7750 (hair nourishing agent),
jojoba oil, vitamin E acetate, Vitaoils nutria (conditioner I) - heated to 75-80°C while stirring continuously until all raw materials are dissolved.

Aqueous phase: purified water, disodium edetate, Esaflor EC3 (viscosity adjuster II), Varisoft 300 (antistatic I) - stirred for the time necessary to homogenise the mixture and heated to 75-80°C. The oil phase was combined with the aqueous phase and homogenised (mechanically) for 5 minutes.

While stirring continuously, it was cooled to 40-42°C and Dexpanthenol (conditioner II), Keracyn (conditioner III), Rep hair (conditioner IV), Menthe Fleur LBPR33361/M fragrance composition, Filcortex (conditioner VII), Dermosoft^{®} OMP (solvent, softener) were added, - stirred and homogenised for 5 minutes until the product cooled to 25-30°C.

The product prepared according to the P1 formulation was characterised by an excessively thick consistency. Preliminary tests showed a poor conditioning effect on the hair. Therefore, another technological trial P2 was prepared.

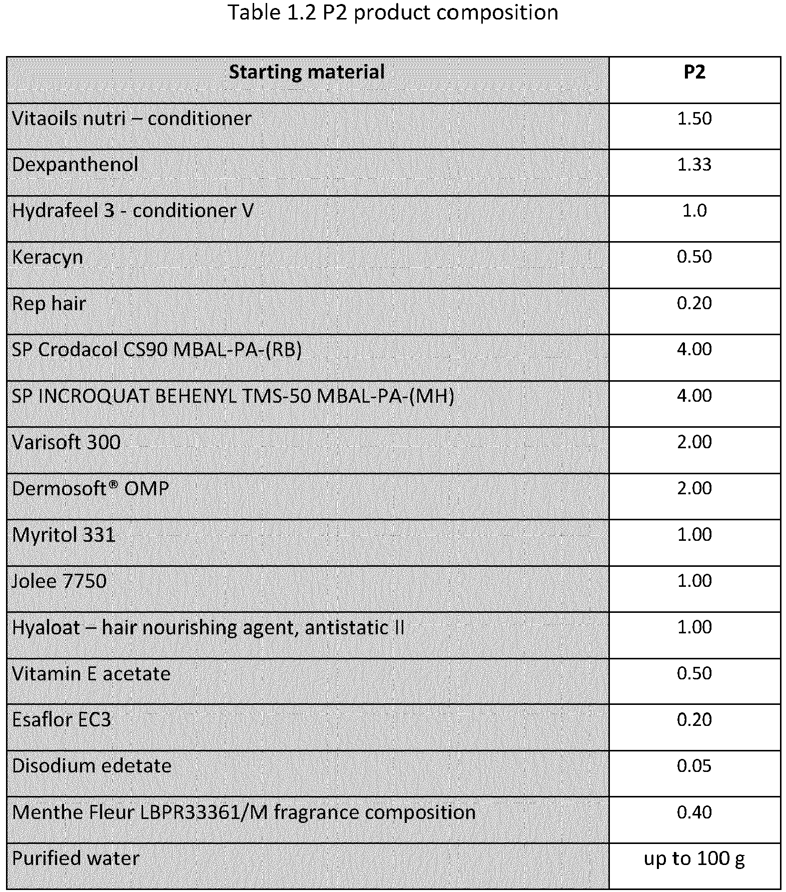

Preparation description:
Oil phase: SP Crodacol CS90 MBAL-PA-(RB) (emulsifier I, emulsion stabilizer viscosity adjuster),
SP INCROQUAT BEHENYL TMS-50 MBAL-PA-(MH) (hair nourishing agent, antistatic, viscosity adjuster I), Myritol 331 (emulsifier II, hair nourishing agent), Jolee 7750 (hair nourishing agent),
Vitaoils nutria (conditioner I), vitamin E acetate - heated to 75-80°C while stirring continuously until all raw materials are dissolved.

Aqueous phase: purified water, disodium edetate, Esaflor EC3 (viscosity adjuster II), Varisoft 300 (antistatic I), Hydrafeel 3 (conditioner V) - stirred for the time necessary to homogenise the mixture and heated to 75-80°C. The oil phase was combined with the aqueous phase and homogenised for 5 minutes.

While stirring continuously, it was cooled to 40-42°C and Dexpanthenol (conditioner II), Keracyn (conditioner III), Dermosoft^{®} OMP (solvent, softener), Rep hair (conditioner IV), Menthe Fleur LBPR33361/M fragrance composition were added, homogenised for 5 minutes and stirred until the product cooled to 25-30°C.

Unexpectedly, it turned out that the product prepared according to the P2 formulation met the requirements in terms of cosmetic formulation, but during stability testing it was found to be inadequate due to significant changes in the pH of the product. Consequently, further trials were undertaken to provide proper product stability. The next stage of the work was preparation of the P3 trial.

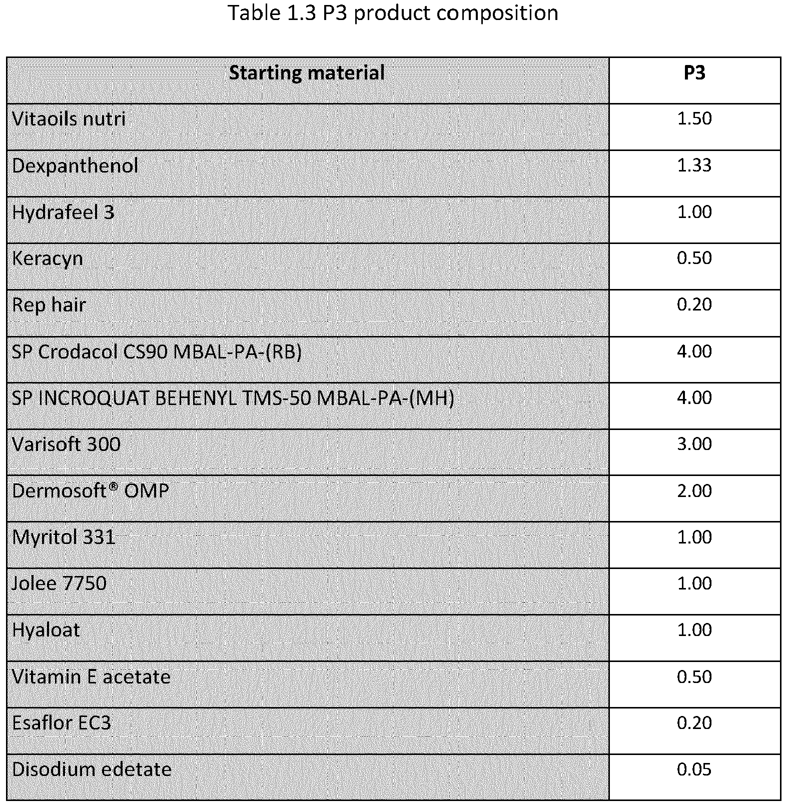

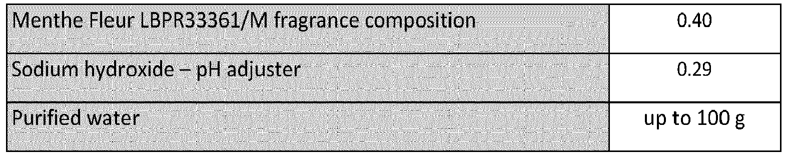

Oil phase: SP Crodacol CS90 MBAL-PA-(RB) (emulsifier I, emulsion stabilizer viscosity adjuster), SP INCROQUAT BEHENYL TMS-50 MBAL-PA-(MH) (hair nourishing agent, antistatic, viscosity adjuster I), Myritol 331 (emulsifier II, hair nourishing agent), Jolee 7750 (hair nourishing agent), Vitaoils nutria (conditioner I), vitamin E acetate - heated to 75-80°C while stirring continuously until all raw materials are dissolved.

Aqueous phase: purified water, disodium edetate, Esaflor EC3 (viscosity adjuster II), Varisoft 300 (antistatic I) - stirred for the time necessary to homogenise the mixture and heated to 75-80°C. The oil phase was combined with the aqueous phase and homogenised for 5 minutes.

Dexpanthenol (conditioner II), Keracyn (conditioner III), Rep hair (conditioner IV), Hydrafeel 3 (conditioner V), Dermosoft^{®} OMP (solvent, softener), Menthe Fleur LBPR33361/M fragrance composition were added at 40-42°C, homogenised and stirred for 5 minutes.

Sodium hydroxide (pH adjuster) in the form of a 10% aqueous solution was then added and stirred until the product cooled to 28-30°C.

The product prepared according to the P3 formulation still showed poor stability during storage. The pH of the product was changing. Consequently, further trials were undertaken to provide proper stability of the product. The next stage of the work was preparation of the P4 trial.

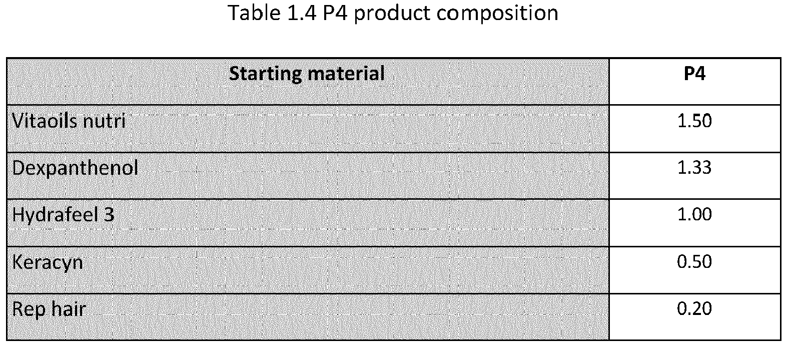

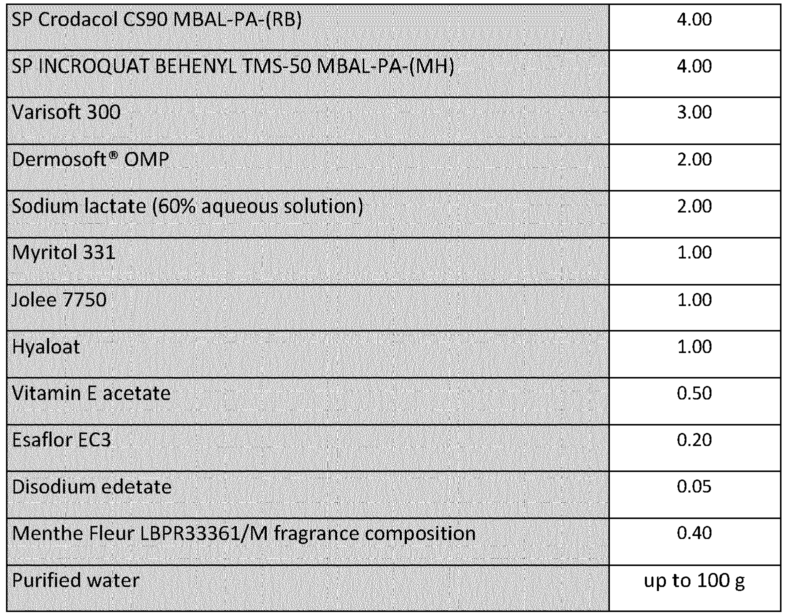

Preparation description:
Oil phase: SP Crodacol CS90 MBAL-PA-(RB) (emulsifier I, emulsion stabilizer viscosity adjuster),
SP INCROQUAT BEHENYL TMS-50 MBAL-PA-(MH) (hair nourishing agent, antistatic, viscosity adjuster I), Myritol 331 (emulsifier II, hair nourishing agent), Jolee 7750 (hair nourishing agent),
Vitaoils nutria (conditioner I), vitamin E acetate - heated to 75-80°C while stirring continuously until all raw materials are dissolved.

Aqueous phase: purified water, disodium edetate, Esaflor EC3 (viscosity adjuster II), Varisoft 300 (antistatic I) - stirred for the time necessary to homogenise the mixture and heated to 75-80°C. The oil phase was combined with the aqueous phase and homogenised for 5 minutes.

Dexpanthenol (conditioner II), Keracyn (conditioner III), Rep hair (conditioner IV), Hydrafeel 3 (conditioner V), Menthe Fleur LBPR33361/M fragrance composition, Dermosoft^{®} OMP (solvent, softener) were added at 40-42°C, homogenised and stirred for 5 minutes.

Sodium lactate (60% aqueous solution) was then added and stirred until the product cooled to 25-30°C.

Unexpectedly, it turned out that the use of sodium lactate provided the stability for this formulation, but an issue with the smell of the cosmetic emerged. Work was undertaken to obtain a formulation that would not change the odour during product storage. The next stage of work involved preparation of a technological trial according to the P5 formulation.

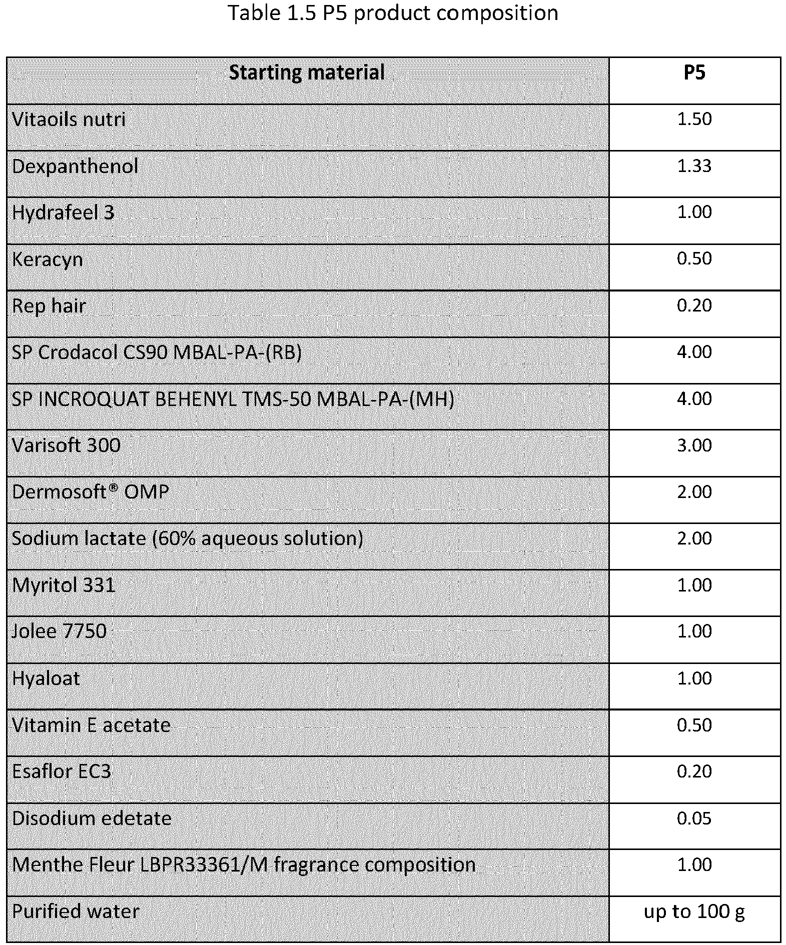

Preparation description:
Oil phase: SP Crodacol CS90 MBAL-PA-(RB) (emulsifier I, emulsion stabilizer viscosity adjuster),
SP INCROQUAT BEHENYL TMS-50 MBAL-PA-(MH) (hair nourishing agent, antistatic, viscosity adjuster I), Myritol 331 (emulsifier II, hair nourishing agent), Jolee 7750 (hair nourishing agent I), Vitaoils nutria (conditioner I), vitamin E acetate - heated to 75-80°C while stirring continuously until all raw materials are dissolved.

Aqueous phase: purified water, disodium edetate, Esaflor EC3 (viscosity adjuster II), Varisoft 300 (antistatic I) - stirred for the time necessary to homogenise the mixture and heated to 75-80°C. The oil phase was combined with the aqueous phase and homogenised for 5 minutes.

Dexpanthenol (conditioner II), Keracyn (conditioner III), Rep hair (conditioner IV), Hydrafeel 3 (conditioner V), Menthe Fleur LBPR33361/M fragrance composition, Dermosoft^{®} OMP were added at 40-42°C, homogenised and stirred for 5 minutes.

Sodium lactate (60% aqueous solution) was then added and stirred until the product cooled to 25-30°C.

The product prepared according to the P5 formulation unexpectedly had an acceptable and stable odour during storage. However, the product was found to have too little effect on hair conditioning. The next stage of the experiment was to prepare the sample according to the P6 formulation.

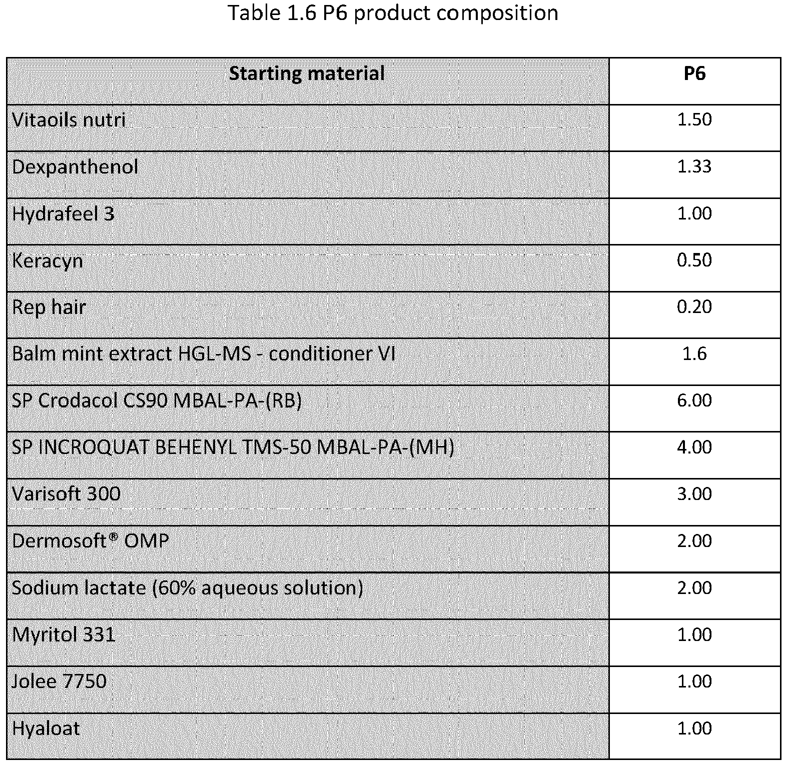

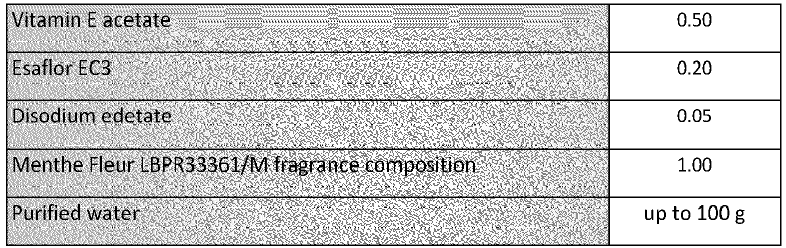

Preparation description:
Oil phase: SP Crodacol CS90 MBAL-PA-(RB) (emulsifier I, emulsion stabilizer viscosity adjuster),
SP INCROQUAT BEHENYL TMS-50 MBAL-PA-(MH) (hair nourishing agent, antistatic, viscosity adjuster I), Myritol 331 (emulsifier II, hair nourishing agent), Jolee 7750 (hair nourishing agent),
Vitaoils nutria (conditioner I), vitamin E acetate - heated to 75-80°C while stirring continuously until all raw materials are dissolved.

Aqueous phase: purified water, disodium edetate, Esaflor EC3 (viscosity adjuster II), Varisoft 300 (antistatic I), Hyaloat - stirred for the time necessary to homogenise the mixture and heated to 75-80°C. The oil phase was combined with the aqueous phase and homogenised for 5 minutes.

Dexpanthenol (conditioner II), Keracyn (conditioner III), Rep hair (conditioner IV), Hydrafeel 3 (conditioner V), Balm mint extract HGL-MS (conditioner VI), Menthe Fleur LBPR33361/M fragrance composition, Dermosoft^{®} OMP (solvent, softener) were added at 40-42°C, homogenised and stirred for 5 minutes.

Sodium lactate (60% aqueous solution) was then added and stirred until the product cooled to 25-30°C.

Unexpectedly, it was found that the product prepared according to the above formulation (P6) had adequate rheological properties and was stable.

The product according to the P6 formulation was characterized by acceptable application qualities and became the starting point for further studies: the selection of a preservative additive and the application and instrumental testing.

### Example 2: Determining the product preservation method

It is routine for cosmetic products to contain a preservative in the formulation. However, during the research work for this product, a formulation was prepared without the addition of additional preservatives: Trial P6.

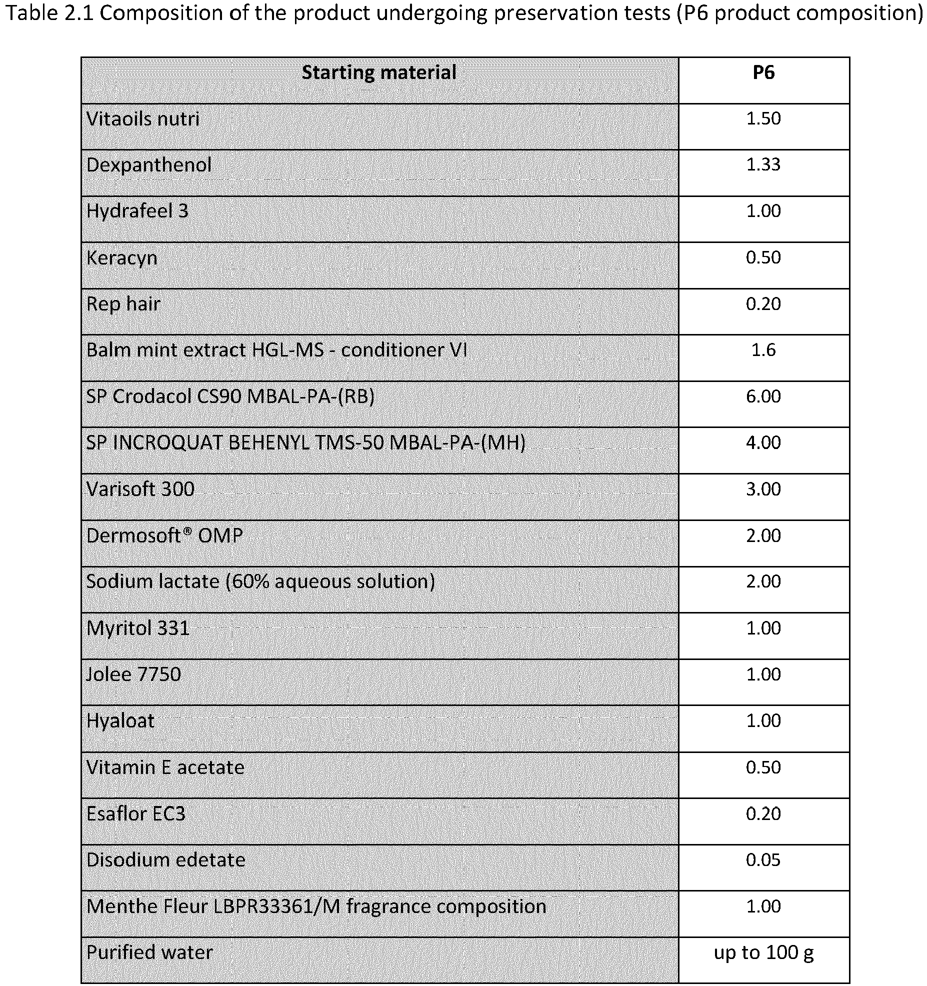

The above P6 formulation was tested for preservation efficacy. An experiment was performed to confirm the efficacy and evaluate the preservation of the cosmetic product. The experiment was performed according to the PN-EN ISO 11930:2012 international standard and consisted of a single introduction of test strains of *Escherichia coli, Staphylococcus aureus, Pseudomonas aeruginosa, Candida albicans* and *Aspergillus brasiliensis* to simulate secondary contamination of the product by a consumer. Inoculated samples were incubated at 22°C in a darkened area. For each strain, the amount of colony-forming microorganisms was tested at 7, 14 and 28 days after inoculation. The test showed that the amount of microorganisms found (in cfu/g) was below the limit of quantification for each of the tested strains, and the reduction values on a logarithmic scale exceeded the minimum values necessary to meet Criterion A specified in the above-mentioned standard. Testing of the cosmetic sample proved that it meets Criterion A specified in PN-EN ISO 11930:2012, i.e. it is protected against the growth of microorganisms that may be a hazard to the user.

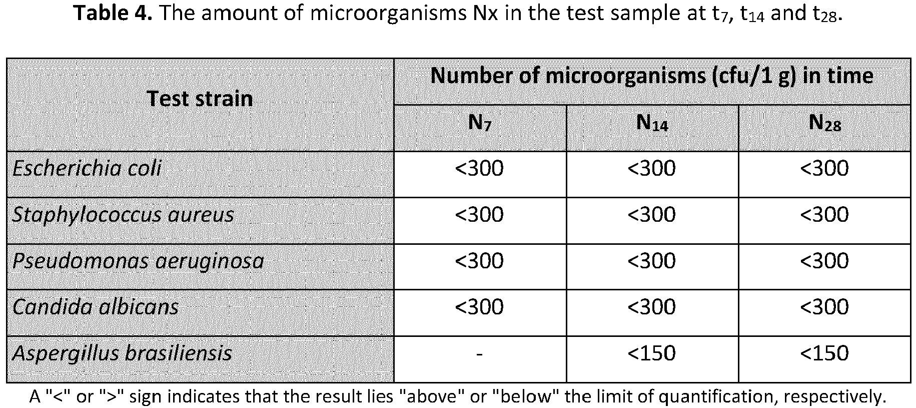

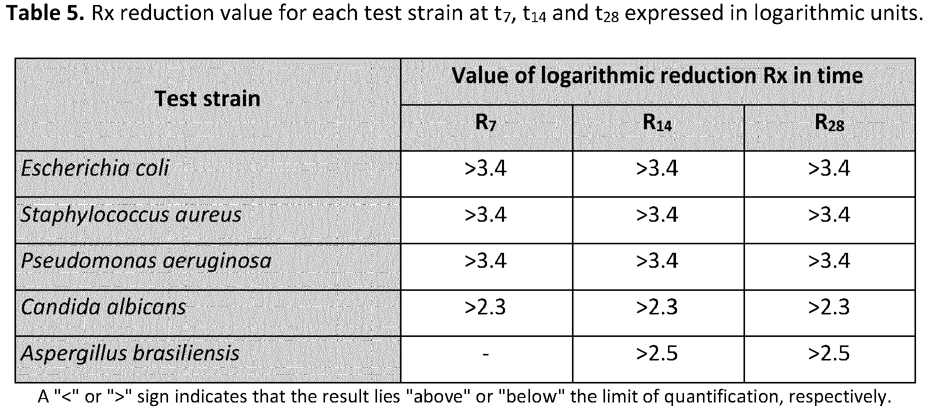

Unexpectedly, it turned out that the tested P6 formulation passed the preservation test despite the lack of any further preservative added. Surprisingly, the P6 trial conformed to the PN-EN ISO 11930:2012 standard and met Criterion A, i.e. it is protected against the growth of microorganisms that may be a hazard to the user. Due to the above, further formulation work to add preservatives to the formulation developed above was abandoned.

### Example 3: In-use testing of the cosmetic product

The product prepared as described (according to the claimed composition - i.e. P6 formulation) was subjected to in-use tests, the purpose of which was, among other, to evaluate the skin tolerance of the applied product among volunteers.

The in-use test was performed with 30 healthy volunteers under home conditions during 4 weeks of regular, repeated application. The study showed good skin tolerance of the product during the application period when applied as intended and following the instructions of use. There were no symptoms indicating intolerance or hypersensitivity to any of the product ingredients. As expected, the volunteers overwhelmingly confirmed the product's intended properties in terms of facilitating hair manageability, improving hair softness and silkiness, improving hair condition, improving hair detangling, less prone to frizz, not weighing hair down, improving retention of healthy hair appearance, maintaining hair shine, improving hair appearance, hair regeneration sensation and better smoothing of hair.

Unexpectedly, most of the respondents found that the applied product protected hair colour from losing its intensity, made hair static-free and that the application of the cosmetic protected hair from damage, e.g. during combing and styling. It was also unexpectedly found that the product keeps hair fresh for a long time.

The full results of the study conducted:
- Does hair become more supple and flexible? (80% positive responses)
- Does the product make hair easier to style? (87% positive responses)
- Does the hair become static-free after using the product? (83% positive responses)
- Does the product moisturise the hair? (97% positive responses)
- Does the product have a long-lasting moisturising effect? (77% positive responses)
- Does the product make the hair less brittle? (80% positive responses)
- Does the hair stay fresh for a long time after applying the mask? (70% positive responses)
- Does the hair become softer and silkier after using the product? (93% positive responses)
- Does regular use of the product improve hair condition? (87% positive responses)
- Does regular use of the product make the hair easier to detangle and less prone to tangling? (93% positive responses)
- Does regular use of the product make the hair less prone to frizz? (87% positive responses)
- Is the product light and does it not weigh down the hair? (93% positive responses)
- Does the product make the hair more manageable? (83% positive responses)
- Does the product reduce split ends? (80% positive responses)
- Does the product reduce hair static electricity? (83% positive responses)
- Does the product protect hair colour from losing its intensity? (73% positive responses)
- Does the product help to preserve healthy hair appearance? (93% positive responses)
- Does the product improve the hair appearance after a single application? (73% positive responses)
- Does the product keep the hair shiny? (93% positive responses)
- Does the product make the hair shinier after use? (100% positive responses)
- Does the product give the sensation of regenerated hair? (93% positive responses)
- Does the product smoothen the hair? (93% positive responses)
- Does the product give the sensation of strengthening the hair? (90% positive responses)
- Does the product nourish even very damaged and demanding hair? (80% positive responses)
- Does the mask protect the hair from the negative effects of heat? (e.g. hair dryer or hair straightener) (70% positive responses)
- Does the product protect the hair from damage, e.g. during combing and styling? (83% positive responses)

### Example 4. Instrumental testing of the cosmetic product

Testing of the density and condition of the hair and epidermis was performed on 8 volunteers, on healthy skin without irritation, using the ARAMO ASW200 hair testing device. The test was carried out at 18-22°C and 45-55% humidity. The volunteers were instructed not to use any antihistamines or other pharmacological agents that could affect the test result. The effect of the product is considered confirmed when the result is confirmed in more than 50% of the test participants.

On the basis of the testing performed, it was found:
a) with regard to the condition of follicular orifices of the hair:
   - the product regenerated the hair in 100% of the volunteers after four weeks of regular application,
   - the product smoothened the hair in 100% of the volunteers after four weeks of regular application,
   - the product strengthened hair in 100% of the volunteers after four weeks of regular application,
b) in relation to the condition of the hair ends:
   - the product reduced split ends in 100% of the volunteers after four weeks of regular application.

Unexpected results were also observed during the study. Based on the analysis of the condition of follicular orifices of the hair, it unexpectedly became apparent that the product had a positive effect on the condition of the hair by not clogging the follicular orifices, making them cleaner. Unblocking the follicular orifices results in adequate growth of new hair and proper scalp function. In addition, based on the analysis of the condition of the hair ends, it was unexpectedly found that the product had a positive effect on the condition of the hair ends, the overall examination of the hair ends confirmed less splitting and fraying of the hair structure at the ends.

## Claims

1. A cosmetic cement composition for hair restoration, **characterised in that** it contains 1.5% of conditioner I (% by weight, amount per 100 g of the product), 1.33% by weight of conditioner II, 0.5% by weight of conditioner III, 0.2% to 0.3% by weight of conditioner IV, 4% to 6% by weight of emulsifier I, 3% to 4% by weight of viscosity adjuster I, 1% to 4% by weight of antistatic I, 2% by weight of softener, 1% by weight of emulsifier II, 1% by weight of hair nourishing agent, 0.5% by weight of vitamin E acetate, 0.2% by weight of viscosity adjuster II, 0.05% by weight of disodium edetate, preferably the composition contains 1.5% by weight of conditioner VII, preferably the composition contains 0.29% to 2% by weight of pH adjuster, preferably the composition contains 1% by weight of antistatic II, preferably the composition contains 1.6% by weight of conditioner VI, preferably the composition contains 1% by weight of jojoba oil.

2. The composition of claim 1, **characterised in that** it contains 1.5% of conditioner I (% by weight, amount per 100 g of the product), 1.33% by weight of conditioner II, 0.5% by weight of conditioner III, 0.2% to 0.3% by weight of conditioner IV, 4% to 6% by weight of emulsifier I, 3% to 4% by weight of viscosity adjuster I, 1% to 4% by weight of antistatic I, 2% by weight of softener, 1% by weight of emulsifier II, 1% by weight of hair nourishing agent, 0.5% by weight of vitamin E acetate, 0.2% by weight of viscosity adjuster II, 0.05% by weight of disodium edetate, 1.6% by weight of conditioner VI, preferably the composition contains 0.29% by weight of sodium hydroxide as a pH adjuster, preferably the composition contains 1% by weight of antistatic II.

3. The composition of claim 1, **characterised in that** it contains 1.5% of conditioner I (% by weight, amount per 100 g of the product), 1.33% by weight of conditioner II, 0.5% by weight of conditioner III, 0.2% to 0.3% by weight of conditioner IV, 4% to 6% by weight of emulsifier I, 3% to 4% by weight of viscosity adjuster I, 1% to 4% by weight of antistatic I, 2% by weight of softener, 1% by weight of emulsifier II, 1% by weight of hair nourishing agent, 0.5% by weight of vitamin E acetate, 0.2% by weight of viscosity adjuster II, 0.05% by weight of disodium edetate, 1.6% by weight of conditioner VI, 2% by weight of lactic acid solution (60% aqueous solution) as pH adjuster, 1% by weight of antistatic II.

4. The composition of any of claims 1 to 3, **characterised in that** it contains a fragrance composition in an amount of 1% by weight of the composition.

5. The composition of any of the preceding claims, **characterised in that** it contains water up to 100% by weight of the composition.

6. The composition of claim 1, **characterised in that** the pH adjuster is selected from the group consisting of: 60% (by weight) aqueous solution of sodium lactate or 10% (by weight) aqueous solution of sodium hydroxide.

7. A method for obtaining the cosmetic cement composition for hair restoration as defined in claim 1, comprising preparation of an oil phase, an aqueous phase, a combination of the oil phase and the aqueous phase, **characterised in that**
a) the oil phase is prepared by mixing emulsifier I, viscosity adjuster I, emulsifier II, hair nourishing agent I, and the resulting mixture is heated to a temperature between 75°C and 80°C and stirred,
b) the aqueous phase is prepared by mixing water, disodium edetate, viscosity adjuster II, antistatic I, and the resulting mixture is stirred and heated to a temperature between 75°C and 80°C,
c) the oil phase and the aqueous phase are then combined and the combined phases are homogenised for 5 minutes, and the homogenised mixture is cooled to a temperature between 40° and 42°C and conditioner I, conditioner II, conditioner III, conditioner IV, vitamin E acetate, softener are added, preferably conditioner VII is added, preferably conditioner V is added, preferably conditioner VI is added and the resulting mixture is homogenised for 5 min, preferably the mixture is cooled to a temperature between 25°C and 30°C, preferably a pH adjuster is added.

8. The method of claim 7, **characterized in that** in step c), after combining the oil phase and the aqueous phase, the combined phases are homogenised for 5 minutes, and the homogenised mixture is cooled to a temperature between 40°C and 42°C and conditioner I, conditioner II, conditioner III, conditioner IV, vitamin E acetate, conditioner V, softener are added to it and the resulting mixture is homogenised for 5 minutes, and a pH adjuster is added, and the mixture is cooled to a temperature between 25°C and 30°C.

9. The method of claim 7, **characterized in that** in step c), after combining the oil phase and the aqueous phase, the combined phases are homogenised for 5 minutes, and the homogenised mixture is cooled to a temperature between 40° and 42°C and conditioner I, conditioner II, conditioner III, conditioner IV, vitamin E acetate, conditioner V, conditioner VI, softener are added to it and the resulting mixture is homogenised for 5 minutes, a pH adjuster is added, and the mixture is cooled to between 25°C and 30°C.

10. The method of any of claims 7 to 9, **characterised in that** in step c), a pH adjuster is added after homogenisation and before cooling, the pH adjuster being selected from the group comprising: 60% (by weight) sodium lactate aqueous solution or 10% (by weight) sodium hydroxide aqueous solution, preferably 60% (by weight) sodium lactate aqueous solution.

11. The method of any of claims 7 to 9, **characterised in that** a fragrance composition is added in step c).

12. The method of claim 7, **characterised in that** the mixture in step a) is stirred until the components of the oil phase are dissolved.

13. The method of claim 7, **characterised in that** the mixture in step b) is stirred until the components of the aqueous phase are dissolved.
